# EUROPÄISCHE PATENTSCHRIFT

(11) **EP 0 898 706 B1**
(45) Veröffentlichungstag und Bekanntmachung des Hinweises auf die Patenterteilung: **12.03.2003**
(21) Anmeldenummer: 97922816.0
(22) Anmeldetag: 26.03.1997
(51) Int. Cl.: G01N 33/483

(54) **GERÄT ZUR HÖCHSTEMPFINDLICHEN MAGNETISCHEN DETEKTION VON ANALYTEN**
DEVICE FOR HIGHLY SENSITIVE MAGNETIC DETECTION OF ANALYTES
APPAREIL SERVANT A LA DETECTION MAGNETIQUE, AVEC UNE SENSIBILITE MAXIMUM, D'ANALYTES

(30) Priorität: 18.04.1996 DE 19615254
(43) Veröffentlichungstag der Anmeldung: 03.03.1999
(73) Patentinhaber: INSTITUT FÜR DIAGNOSTIKFORSCHUNG GmbH AN DER FREIEN UNIVERSITÄT BERLIN, 14050 Berlin (DE)
(72) Erfinder: KOCH, Hans, D-12159 Berlin (DE); MATZ, Hartmut, D-12157 Berlin (DE); KÖTITZ, Roman, D-13189 Berlin (DE); DRUNG, Dietmar, D-13583 Berlin (DE); TRAHMS, Lutz, D-12103 Berlin (DE); WEITSCHIES, Werner, D-12205 Berlin (DE); SEMMLER, Wolfhard, D-13467 Berlin (DE)
(74) Vertreter: KOHLER SCHMID + PARTNER
(86) Internationale Anmeldenummer: DE9700611
(87) Internationale Veröffentlichungsnummer: WO97040377

(56) Entgegenhaltungen:
- WO-A-96/23227
- WO-A-96/27133
- US-A- 4 913 883
- US-A- 5 486 457
- PATENT ABSTRACTS OF JAPAN vol. 15, no. 507 (P-1291), 20.Dezember 1991 & JP 03 220442 A (TDK CORP), 27.September 1991, in der Anmeldung erwähnt

## Beschreibung

Die Erfindung betrifft eine Vorrichtung zum qualitativen und/oder quantitativen Nachweis von Analyten in einer insbesondere auch biologischen Meßprobe mittels Rezeptor-Ligand-Bindungen mit einer Aufmagnetisierungseinrichtung zur Erzeugung eines Magnetfeldes am Ort der Meßprobe und mit einer Detektionseinrichtung zur Messung von magnetischen Eigenschaften der Meßprobe.

Ähnliche Geräte sind indirekt durch Beschreibung eines auf den entsprechenden Geräten durchgeführten Meßverfahrens aus der JP 63 09 0765-A2 bekannt.

Geräte zur Messung von Rezeptor-Ligand-Bindungen beruhen auf der Vermessung von Signalen, die durch signalerzeugende Label, mit denen strukturspezifische Substanzen markiert sind, generiert werden. Die bisher empfindlichsten Geräte beruhen auf der Detektion radioaktiv markierter Substanzen (RadioImmunoAssay, RIA). Die Verwendung radioaktiver Label bringt offensichtliche Nachteile mit sich wie z.B. Probleme bei der Lagerung und Beseitigung der radioaktiven Substanzen. Außerdem erfordern derartige Geräte eine Einrichtung zur Trennung der gebundenen von den ungebundenen Labeln. Ohne diese Separation ist eine quantitative Aussage in der Regel nicht möglich.

Alternative Geräte basieren auf der optischen Vermessung von Agglutinations-, Fluoreszenz-, und Farbreaktionen (FIA, ELISA). Hier handelt es sich im wesentlichen um Photodetektoren. Auch bei diesen Verfahren ist in der Regel eine Separation für eine quantitative Aussage notwendig. Andererseits gibt es eine große Anzahl von Meßgeräten zur Bestimmung magnetischer Eigenschaften von Proben, die bisher allerdings zumeist nicht zur direkten Detektion von Rezeptor-Liganden-Bindungen eingesetzt wurden.

Es sind einige Geräte bekannt, die auf Verfahren beruhen, bei denen magnetische Label eingesetzt werden. Beispielsweise werden durch Anlegen eines Magnetfeldes magnetisch markierte Teilchen bewegt und die Bewegung z.B. mittels Laser nachgewiesen. Desweiteren gibt es Geräte, die auf Verfahren beruhen, bei denen magnetische Label zur Trennung von gebundenen und ungebundenen Bestandteilen verwendet werden. Dagegen gibt es sehr wenige Geräte, die auf der Vermessung der magnetischen Eigenschaften der Meßprobe beruhen.

In JP 6 309 0765 ist ein SQUID-Immunoassay Verfahren beschrieben, das auf magnetisch markierten Antikörpern oder Antigenen beruht. Nach der Antikörper-Antigen Reaktion müssen allerdings die ungebundenen Bestandteile aus der Probe entfernt werden (Separation). Ein dafür geeignetes Gerät muß demnach eine Vorrichtung zur Trennung der gebundenen von den ungebundenen Labeln enthalten. Nach der Trennung wird die Magnetisierung der Probe in Anwesenheit eines magnetischen Feldes gemessen, d.h. die Messung der Magnetisierung findet im Feld statt.

In der US 4,913,883 ist ein Gerät für ein Immuno-Agglutinations-Assay beschrieben. Es beruht auf der Messung der Agglutination von mit magnetischen Partikeln im µm Größenbereich markierten Antikörpern. Dazu enthält das Gerät notwendigerweise eine Einrichtung zur Vereinzelung der Agglutinate und eine Einrichtung zum Transport dieser Agglutinate mittels eines Flüssigkeitsstroms durch die Detektionseinrichtung.

In der JP 3-220442 A ist ein Meßverfahren zur Durchführung von Agglutinations-Immunoassays offenbart, bei dem das Ausmaß der antikörpervermittelten Agglutination mittels eines in der Druckschrift offenbarten Verfahrens zur Messung der Teilchengrößen agglomerierter magnetischer Teilchen durchgeführt wird. Die Methode besteht darin, ein Magnetfeld, welches die ortsfeste in flüssiger Form vorliegende Probe durchdringt, zu schalten und die residuale magnetische Flußdichte der agglomerierten magnetischen Teilchen zu messen.

Gemäß der JP 3-220442 A kann die Bestimmung des Ausmaßes der Agglutination ebenso mittels optischer Verfahren zur Bestimmung von Teilchengrößen durchgeführt werden. Insoweit handelt es sich bei dem offenbarten Verfahren ausschließlich um ein Verfahren zur Bestimmung der durch Agglutination entstehenden Teilchengrößen magnetischer Teilchen, das in der beschriebenen Form auch nur auf Agglomerate magnetischer Teilchen anwendbar ist, wobei die Teilchengrößen der Agglomerate im Mikrometer-Bereich liegen.

Ein in der US 5 486 457 beschriebenes Meßverfahren dient zur Bestimmung der Beweglichkeit an Zellen gebundener magnetischer Teilchen. Die dort beschriebenen Geräte messen Magnetfelder in Gegenwart eines zum magnetisierenden Feld um 90° gedrehten schwächeren magnetischen Feldes.

Ein in dem Artikel von Valberg et al, Science 1984, Bd. 424, S. 513-516 dargestelltes Meßverfahren basiert auf Magnetfeldmessungen unter Rotation von magnetischen Partikeln mit Teilchengrößen von typischerweise 0.7 µm. Zur Erhöhung der Meßempfindlichkeit wird explizit Lock-In-Technik benutzt. Dies ist ein Modulationsverfahren, bei dem nur schmalbandig das Meßsignal aufgenommen wird.

In dem Artikel von Philo et al, Rev. Sci. Instrum. 1977, Bd. 48, S. 1529-1536 wird ein Verfahren beschrieben, mit dem mit Hilfe von SQUID-Technologie Volumensuszeptibilitäten gemessen werden können. Dort wird explizit der Vorteil von SQUIDs für hochempfindliche Messungen herausgestellt, welcher für zukünftige Meßgeräte von Nutzen sein kann.

In den nicht vorveröffentlichten deutschen Patentanmeldungen DE 195 03 664.6 und DE 195 08 772.0 sind Verfahren und Verbindungen zur magnetorelaxometrischen Detektion von Analyten bzw. zur Detektion von Analyten mittels Remanenzmessung beschrieben. Als magnetorelaxometrische Detektion wird im folgenden die bindungsspezifische Detektion von Analyten in Flüssig- oder Festphasen bezeichnet, die dadurch gekennzeichnet ist, daß ferro- oder ferrimagnetische kolloidale Teilchen als nachzuweisende magnetische Markierung zum Nachweis von Analyten mittels Ligand-Rezeptor-Bindungen eingesetzt werden und die Relaxation ihrer Magnetisierung als Meßgröße bestimmt wird. Als Detektion von Analyten mittels Remanenzmessung im folgenden auch Messung der Bindungsremanenz genannt wird im folgenden die bindungsspezifische Detektion von Analyten in Flüssig- oder Festphasen bezeichnet, die dadurch gekennzeichnet ist, daß stabile oder quasistabile ferro- oder ferrimagnetische Substanzen als nachzuweisende magnetische Markierung zum Nachweis von Analyten mittels Ligand-Rezeptor-Bindungen eingesetzt werden und die Remanenz ihrer Magnetisierung als Meßgröße bestimmt wird. Bei den beiden letzt genannten Verfahren wird
I) die Relaxation (das zeitliche Abklingen der Magnetisierung) der Meßprobe unmittelbar nach Abschalten oder Entfernen des magnetisierenden Feldes oder
II) die frequenzabhängige Magnetisierung der Meßprobe in Gegenwart eines magnetisierenden Feldes oder
III) die bindungsspezifische Remanenz der Meßprobe nach Aufmagnetisieren gemessen.

Dabei ist es wünschenswert,
1. externe Störsignale (z.B. Netzbrummen, Erdmagnetfeldschwankungen) ausreichend zu unterdrücken und möglichst keine internen Störsignale zu erzeugen,
2. auf aufwendige magnetische Abschirmmaßnahmen zu verzichten, sowie
3. einen einfachen und schnellen Probenwechsel durchzuführen.

Um die genannten neuartigen Verfahren ausführen zu können, ist daher ein neuartiges Gerät erforderlich, das durch Messung magnetischer Eigenschaften von Meßproben eine hochempfindliche quantitative und qualitative Detektion von Rezeptor-Liganden-Bindungen ermöglicht.

Da bisher keine derartigen Geräte bekannt sind, die sich für eine routinemäßige, kostengünstige Durchführung der oben beschriebenen neuartigen Meßverfahren eignen, ist es Aufgabe der vorliegenden Erfindung, Vorrichtungen der eingangs beschriebenen Art vorzustellen, mit denen die neuartigen Meßverfahren durchgeführt werden können.

Gemäß einem ersten Aspekt der vorliegenden Erfindung wird diese Aufgabe dadurch gelöst, daß bei einer Vorrichtung mit den eingangs beschriebenen Merkmalen die Aufmagnetisierungseinrichtung räumlich derart zu der Detektionseinrichtung angeordnet ist, daß das von der Aufmagnetisierungseinrichtung am Ort der Aufmagnetisierung erzeugte Magnetfeld am Ort, den die Meßprobe während der Messung einnimmt, um mindestens einen Faktor 10, vorzugsweise um einen Faktor 1000 oder mehr abgeschwächt ist.

Bei einem zweiten Aspekt der vorliegenden Erfindung wird die obige Aufgabe dadurch gelöst, daß eine Schalteinrichtung vorgesehen ist, die über eine vorgegebene Zeitdauer während der Meßphase der Detektionseinrichtung, das Magnetfeld der Aufmagnetisierungseinrichtung am Ort der Meßprobe abschalten kann, und daß eine Vorrichtung zur Bewegung der Meßprobe während der Meßphase der Detektionseinrichtung vorgesehen ist. Damit ist auch eine Remanenzmessung ohne zeitlich veränderliches Aufmagnetisierungsfeld möglich. Durch Mittelung bzw. Filterung kann bei dieser Ausführungsform das Signal-zu-Rausch-Verhältnis entscheidend verbessert werden. Außerdem ist damit eine Vereinfachung und Automatisierung des Probentransports sowie der automatische Betrieb des gesamten Meßsystems bei großen anfallenden Probenstückzahlen möglich.

Wesentlich dabei ist, daß bei einem entsprechenden Gerät die Meßprobe zwar aufmagnetisiert wird, die Vermessung der magnetischen Eigenschaften der Meßprobe aber in Abwesenheit bzw. bei hinreichender Abschwächung des magnetisierenden Feldes durchgeführt wird (Meßphase).

Dies kann erfindungsgemäß entweder durch eine räumliche Trennung der Aufmagnetisierungseinrichtung von der Detektionseinrichtung oder durch eine zeitliche Trennung des Magnetisierungsvorgangs von der Messung erreicht werden.

Bei Verwendung der erfindungsgemäßen Vorrichtungen zur Durchführung der oben beschriebenen neuartigen Verfahren besteht ein entscheidender Vorteil darin, daß das Meßsignal gebundener magnetischer Marker sich deutlich vom Signal ungebundener magnetischer Marker unterscheiden läßt und somit eine Separation der ungebundenen von den gebundenen Markern nicht notwendig ist. Des weiteren kann die Bindungskinetik ohne Probenwechsel untersucht werden.

Des weiteren ermöglicht eine Vorrichtung gemäß der vorliegenden Erfindung bei Anwendung der genannten neuartigen Verfahren die simultane Bestimmung mehrerer Analyten in einer komplexen Probe (Multianalytassay).

Bei der Vorrichtung gemäß dem ersten Aspekt der Erfindung ermöglicht die räumliche Trennung der Aufmagnetisierungseinrichtung vom Meßort sehr hohe Magnetisierungsfeldstärken, ohne die Detektionseinrichtung zu beeinflussen. Es kann bereits während des Bindungsprozesses magnetisiert werden. Die Probenpräparation kann getrennt vom Meßort, beispielsweise in verschiedenen Labors oder gar in verschiedenen Städten erfolgen. Zur Aufmagnetisierung können auch Permanentmagnete verwendet werden, die keiner Energiezufuhr während der Aufmagnetisierungsphase bedürfen.

Bei der Vorrichtung gemäß dem oben beschriebenen zweiten Aspekt der Erfindung kann das zur Aufmagnetisierung der Meßprobe erforderliche Feld abgeschaltet werden und somit in Abwesenheit des Aufmagnetisierungsfeldes gemessen werden.

Besonders bevorzugt ist eine Ausführungsform der erfindungsgemäßen Vorrichtung, bei der die Detektionseinrichtung eine Vorrichtung zur Messung der Magnetisierung der Meßprobe enthält. Die Magnetisierung ist für derartige Proben diejenige Meßgröße, die am empfindlichsten gemessen werden kann.

Vorteilhaft ist auch eine Ausführungsform, bei der die Detektionseinrichtung eine Vorrichtung zur Messung der Bindungsremanenz des Analyten in der Meßprobe enthält. Die Messung der Bindungsremanenz ermöglicht ein Multianalytassay. Außerdem kann die Probenpräparation getrennt vom Meßort erfolgen, beispielsweise in verschiedenen Labors oder in verschiedenen Städten. Auch diese Meßart erreicht eine hohe Nachweisempfindlichkeit, wobei die Probenpräparation und die Meßdurchführung relativ einfach sind. Die Messung der Bindungsremanenz kann auch für In vivo-Untersuchungen angewendet werden.

Eine In vivo-Anwendung ist auch bei einer Ausführungsform der erfindungsgemäßen Vorrichtung möglich, bei der die Detektionseinrichtung eine Vorrichtung zur magnetorelaxometrischen Detektion enthält.

Diese Vorrichtung ermöglicht kurze Meßphasen, die es beispielsweise zulassen, Reaktionskinetiken mit hoher zeitlicher Auflösung in einer Meßprobe durchzuführen.

Ganz besonders bevorzugt ist eine Ausführungsform, bei der die Detektionseinrichtung mindestens ein SQUID (Superconducting Quantum Interference Device) als Teil des Magnetfeldsensors enthält, weil SQUIDs die empfindlichsten aller derzeit bekannten Magnetfeldsensoren sind.

Bei einer weiteren Ausführungsform enthält die Detektionseinrichtung mindestens eine Induktionsspule als Teil des Magnetfeldsensors. Derartige Induktionsspulen sind einfach aufzubauen, preisgünstig in der Herstellung und bei hohen Frequenzen relativ empfindlich.

Bei einem dritten Aspekt der Erfindung ist eine Schalteinrichtung vorgesehen, die über eine vorgegebene Zeitdauer während der Meßphase der Detektionseinrichtung, das Magnetfeld der Aufmagnetisierungseinrichtung am Ort der Meßprobe abschalten kann, und die Schalteinrichtung umfaßt eine erste Einrichtung zum Ein- und Ausschalten des in der Aufmagnetisierungseinrichtung erzeugten Magnetfeldes sowie eine zweite Einrichtung zum Ein- und Ausschalten der Detektionseinrichtung.

Bei einer ersten Weiterbildung dieser Ausführungsform der Erfindung sind die erste und die zweite Einrichtung unabhängig voneinander schaltbar. Dadurch kann die Messung nach Abschalten des Magnetfeldes und nach geeigneter zeitlicher Verzögerung gestartet werden.

Bei einer alternativen Weiterbildung der obigen Ausführungsform ist die erste Einrichtung mit einer vorgebbaren, starren zeitlichen Korrelation zur zweiten Einrichtung schaltbar. Die geeignete Wahl des Einschaltzeitpunktes ermöglicht bei dieser Ausführungsform eine Ausblendung von Prozessen mit kürzerer Zeitkonstante und damit eine selektive Signalerfassung. Durch das damit ermöglichte frühestmögliche Einschalten der Meßeinrichtung kann außerdem das Signal-zu-Rausch-Verhältnis bei Relaxationsmessungen verbessert werden.

Bevorzugt ist auch eine Weiterbildung der zuletzt genannten drei Ausführungsformen der Erfindung, bei der die erste Einrichtung vorgebbare Feldamplituden und Feldpolaritäten des in der Aufmagnetisierungseinrichtung erzeugten Magnetfeldes erzeugen kann. Damit läßt sich einerseits die Probe besonders gezielt aufmagnetisieren und andererseits durch zeitliche Mittelung über mehrere Perioden das Signal-zu-Rausch-Verhältnis verbessern. Dies kann z. B. durch einen Chopperbetrieb erreicht werden. Diese Ausführungsformen sind besonders zur Durchführung von Multianalytassays geeignet.

Bei einer Weiterbildung der eben genannten Ausführungsform ist vorgesehen, daß die erste Einrichtung vorgebbare zeitliche Amplitudenverläufe und vorgebbare zeitliche Polaritätsverläufe des in der Aufmagnetisierungseinrichtung erzeugten Magnetfeldes erzeugen kann. Dies ermöglicht Remanenzmessungen ohne räumliche Bewegung der Probe. Das Prinzip der Bindungsremanenzmessung kann dabei auch im Falle von In vivo-Messungen angewendet werden. Außerdem sind Multianalytassays möglich. Weiter können konstante bzw. stationäre Störfelder leicht kompensiert werden. Das Signal-zu-Rausch-Verhältnis kann durch Vergleichsmessungen und Mittelungsverfahren weiter verbessert werden. Mit der Vorrichtung ist auch eine Messung der Magnetisierungskurve möglich und schließlich kann das SQUID-System permanent im unten näher beschriebenen FLL-Modus betrieben werden und somit das angelegte Magnetfeld selbst gemessen werden.

Besonders vorteilhaft ist auch eine Ausführungsform der erfindungsgemäßen Vorrichtung, bei der eine Einrichtung zur elektronischen Störsignalunterdrückung vorgesehen ist. Dadurch wird ein noch höheres Signal-zu-Rausch-Verhältnis sowie die Durchführung von unabgeschirmten Messungen möglich. Aufwendige und kostspielige Abschirmvorrichtungen können daher eingespart werden. Außerdem können derartige Geräte in einer nahezu beliebigen Umgebung betrieben werden. Außerdem sind damit die Ergebnisse vom Aufstellungsort weitgehend unabhängig.

Bei einer Weiterbildung dieser Ausführungsform umfaßt die Einrichtung zur elektronischen Störsignalunterdrückung eine Einheit zur adaptiven Filterung. Dies ermöglicht ein noch weiter verbessertes Signal-zu-Rausch-Verhältnis, da Störsignale aktiv unterdrückt werden. Mit der Anpassung auf das Anregungssignal ist außerdem eine höhere Detektionsempfindlichkeit verbunden.

Besonders vorteilhaft ist auch eine Ausführungsform der erfindungsgemäßen Vorrichtung, bei der eine Einrichtung zur Störfeldvektormessung und eine damit verbundene Einrichtung zur entsprechenden Kompensation des mit der Detektionseinrichtung gemessenen Signales und/oder des von der Aufmagnetisierungseinrichtung erzeugten Magnetfeldes vorgesehen ist. Durch Kenntnis der Störfeldrichtung ist eine wesentlich bessere Balancierung der Apparatur möglich. Außerdem kann die Signaländerungsgeschwindigkeit der Sensor-SQUIDs erhöht werden und eine Überprüfung der Homogenität des Anregungsmagnetfeldes wird ermöglicht.

Die oben genannten Ausführungsformen der erfindungsgemäßen Vorrichtung, insbesondere diejenigen, welche zur Messung der Bindungsremanenz oder zur magnetorelaxometrischen Detektion eingerichtet sind, können insbesondere auch für In vivo-Messungen am Menschen oder an Versuchstieren ausgestaltet sein.

Weitere Vorteile der Erfindung ergeben sich aus der Beschreibung und der Zeichnung. Ebenso können die vorstehend genannten und die noch weiter aufgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Die Erfindung ist in der Zeichnung dargestellt und wird anhand eines Ausführungsbeispiels näher erläutert. Es zeigen:
- Fig. 1: eine schematische Längsschnittansicht einer ersten Ausführungsform der erfindungsgemäßen Vorrichtung;
- Fig. 2: das Schema einer räumlichen Verteilung von mehreren in Form einer Matrix angeordneten Meßproben sowie eine zugehörige Sensorzeile;
- Fig. 3: eine schematische Schnittdarstellung einer zweiten Ausführungsform der erfindungsgemäßen Vorrichtung;
- Fig. 4: eine schematische Schnittdarstellung einer dritten Ausführungsform der erfindungsgemäßen Vorrichtung; und
- Fig. 5: eine schematische Schnittdarstellung einer vierten Ausführungsform der erfindungsgemäßen Vorrichtung.

Zur Messung des durch Bindung von magnetisch markierten strukturspezifischen Substanzen erzeugten Magnetfeldes werden folgende Detektoren vorgeschlagen:
1. SQUIDs (sowohl High Tc als auch Low Tc)
2. Induktionsspulen (eventuell in Kombination mit einem magnetischen Kern analog zum Tonkopf bei Magnetbändern)
3. flux gate Sensoren
4. magnetoresistive Widerstände insbesondere GMR Sensoren

Um geringste Mengen von gebundenen Analyten in einer Lösung nachzuweisen bedarf es Magnetfelddetektoren mit sehr kleinen Energieauflösungen. Diese können z. B. mit SQUIDs realisiert werden. Derartige SQUIDs können unter bestimmten Randbedingungen auch bei relativ großen Magnetfeldern betrieben werden und bieten sich durch die Flexibilität entsprechender supraleitender Feldaufnahmespulen als Detektoren an. Gegebenenfalls können diese auch durch andere Detektoren ersetzt werden (siehe oben).

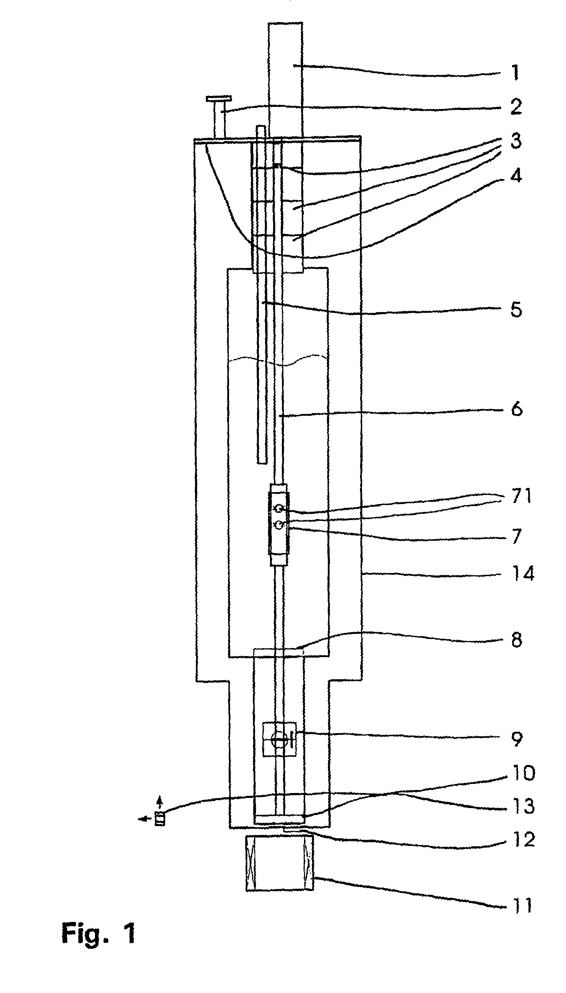

Das zur Lösung der Problemstellung erfindungsgemäß bevorzugte Gerät weist für die Relaxations- bzw. Remanenzmessung der Analyten die gleiche Detektorkonfiguration auf. Meßmethodenspezifische Unterschiede bestehen im wesentlichen in der Art der Aufmagnetisierung der Probe und im Betriebsmodus der Detektoren. In Fig. 1 ist beispielhaft das Prinzipdiagramm einer möglichen erfindungsgemäßen Gerätekonfiguration dargestellt.

Im einzelnen ist in Fig. 1 eine elektronische Schaltung 1 zu erkennen, die im folgenden "FLL-Elektronik" genannt wird, da mit ihr das SQUID in einem geschlossenen Regelkreis (FLL-Mode) betrieben werden kann. Weiterhin ist ein Vakuumstutzen 2, eine Anordnung von Baffles 3, ein Dewardeckel 4, ein Überfüllstutzen 5, eine Sensorhalterung 6, ein magnetisch geschirmter SQUID-Container 7, eine Feldaufnahmespule des Referenzgradiometers 8, ein Vektormagnetometer 9, eine Feldaufnahmespule des Sensorgradiometers 10, eine Anregungsspule 11, eine Meßprobe 12 sowie eine positionsvariable Kompensationsspule 13 dargestellt.

Als Sensoren werden ein oder mehrere SQUIDs 71 eingesetzt. Die Sensoren müssen aufgrund ihres Funktionsprinzips in einem Kryostaten 14 betrieben werden, der die Kühlflüssigkeit (flüssiges Helium LHe beziehungsweise flüssiger Stickstoff LN₂) zur Gewährleistung der Supraleitung aufnimmt. Alternativ kann die Kühlung bei nicht gezeigten Ausführungsformen auch durch eine Kältemaschine gewährleistet werden. Da die Proben zumeist in flüssigem Zustand vorliegen, bedarf es einer thermischen Isolation zwischen SQUID und Probe, die im einfachsten Fall, wie in Fig. 1 dargestellt, durch die Kryostatenwand erfolgen kann. Um eine gute magnetische Kopplung zwischen den Analyten in der Probe und der Feldaufnahmespule des Sensorgradiometers zu gewährleisten, muß der Abstand zwischen diesen minimiert werden, wobei der Abstand vorzugsweise kleiner als der effektive Aufnahmespulendurchmesser sein sollte.

Zur Vermeidung eines Ausfrierens der flüssigen Proben können diese gegebenenfalls auch beheizt werden, z. B. optisch mittels Laser.

Zur Aufmagnetisierung der Meßprobe 12 wird vorzugsweise eine normalleitende Anregungsspule 11 außerhalb des Kryostaten 14 verwendet. Eine supraleitende oder normalleitende Sule innerhalb des Dewars ist ebenfalls einsetzbar. Bei den oben erwähnten neuartigen Verfahren I und III (Messung der Relaxation und Messung der zeitunabhängigen Remanenz der Probe) sollte sich die Probe während der Messung annähernd im magnetfeldfreien Raum befinden. Dies kann durch Kompensationsmaßnahmen, die im weiteren noch näher beschrieben werden, erfolgen. Bei Verwendung eines "flux gate Sensors" oder beweglicher Feldaufnahmespulen als Referenzsensor kann das Absolutfeld in der Umgebung der Probe bestimmt werden und durch entsprechende Kompensationsspulen 13, die vom Aufstellungsort abhängen, kompensiert werden. (Kompensation des Störfeldes)

Bei Verfahren II (Messung der frequenzabhängigen Magnetisierung der Probe) wird die Probe einem magnetischen Wechselfeld ausgesetzt, welches in seiner Frequenz variiert werden kann. Das Anregungsfeld sollte dabei im Bereich des Meßvolumens homogen sein.

Durch eine geeignete Feldaufnahmespule (Antenne) wird das Meßsignal in den SQUID Sensor eingekoppelt. Diese Antennenkonfiguration wird zweckmäßigerweise als Planargradiometer aus zwei sich kompensierenden Feldspulen möglichst gleicher Geometrie ausgeführt, wobei die Spulen in Reihe oder parallel geschaltet sein können. Die Meßprobe wird zweckmäßigerweise so angeordnet, daß diese von einer der Spulen umschlossen wird oder sich direkt unter ihr befindet, wie es in Fig. 1 gezeigt ist. Zur Erzielung einer sehr hohen Meßempfindlichkeit ist ein minimaler Abstand zwischen den aktiven Bereichen der Meßprobe und der Antennenspule erforderlich. Dies kann durch eine minimierte Kryostatenwandstärke in diesem Bereich erreicht werden. Der Kryostat kann außerhalb des Probenbereiches eine wesentlich dickere Wandstärke aufweisen.

Die Balance des Sensorgradiometers 10 (d.h. Abweichung der effektiven gerichteten Flächen der beiden Spulen bezogen auf die effektive Fläche) aufgrund ihrer Geometrie sowie der Symmetrie des magnetisierenden Feldes ist von essentieller Bedeutung und kann durch die Einkopplung eines Kompensationsfeldes mittels der zusätzlichen Kompensationsspule 13, die synchron mit der Anregungsspule 11 angesteuert wird, verbessert werden. Der Abgleich des Sensorgradiometers 10 im magnetisierenden Feld kann z. B. durch ein Potentiometer rechnergesteuert erfolgen oder fest justiert werden. In einer Leermessung ohne Probe wird die Anregungsspule, vorzugsweise mit einem Wechselstrom gespeist und der Strom durch die Kompensationsspule 13 solange nachgeregelt bis sich am SQUID-Ausgang ein minimales Signal ergibt. Diese Einstellung wird für die Probenvermessung beibehalten. Gegebenenfalls muß ebenfalls eine Phasenkorrektur erfolgen.

Durch diese Maßnahmen werden sowohl Abweichungen in der Antennenspulengeometrie als auch Unsymmetrien des magnetisierenden Feldes in Bezug auf die Feldaufnahmespulen maximal unterdrückt und das Meßsignal wird praktisch unabhängig von der zeitlichen Variation des magnetisierenden Feldes. Treten während der Messungen unerwartete Balanceverschiebungen auf oder ist die Kompensation nicht ausreichend präzise, kann dieser Fehler z. B. durch Positionieren der Meßprobe 12 unter der anderen Spule des Sensorgradiometers 10 behoben werden. In einigen Fällen kann es zweckmäßig sein, das Sensorgradiometer 10 nicht vollständig zu balancieren, um ein Maß für die Magnetisierungsstärke zu erhalten.

Durch diese Maßnahmen wird ermöglicht:
1. die schwache frequenzabhängige Magnetisierung der Probe in Gegenwart eines starken Wechselfeldes zu messen und
2. die Relaxation der Probe unmittelbar nach Abschalten des magnetisierenden Feldes zu messen, da das Abschalten des Feldes selbst nicht mehr zum Meßsignal beiträgt.

Zu berücksichtigen ist, daß die SQUID-Sensoren 71 (bzw. die Josephson-Kontakte) selbst magnetfeldabhängig sind und sich deren Arbeitspunkt durch zu große Magnetisierungsfelder unkontrollierbar verschieben kann. Um trotzdem ein schnelles Einschwingen der FLL-Elektronik 1 bzw. einen kontinuierlichen Meßbetrieb zu gewährleisten, kann das SQUID räumlich von der Feldaufnahmespule getrennt angeordnet werden, wie es in Fig. 1 angedeutet ist. Dazu kann das SQUID in einiger Entfernung innerhalb einer supraleitenden Abschirmung orthogonal zur Magnetisierungsrichtung in dem Container 7 positioniert werden. Die Verbindung zwischen Feldaufnahmespule und SQUID kann z. B. durch supraleitende vertwistete Zuleitungen, die gegebenenfalls magnetisch geschirmt werden (Bleikapillare), ausgeführt werden. Zusätzlich kann eine "Demagnetisierungsspule", die um den supraleitenden Schirm (SQUID-Container 7) des SQUID angeordnet ist und von einem Teil des Erregerfeldstromes durchflossen wird, angebracht werden. Deren Aufgabe besteht in der Reduzierung der Feldstärke in der Umgebung der Abschirmung und der Verringerung des durch die Aufmagnetisierung erzeugten Streufeldes. Supraleitender Schirm und Demagnetisierungsspule sollten so angeordnet werden, daß die Feldverzerrungen am Ort der Feldaufnahmespulen und der Meßprobe minimal sind.

Um die höchste Feldauflösung des Meßsystemes und einen linearen Zusammenhang zwischen Ausgangsgröße und gemessenem Magnetfeld zu erreichen, sollte das SQUID, wie bereits oben erwähnt, in einem geschlossenen Regelkreis betrieben werden (FLL-Elektronik 1). Dazu wird das SQUID als Nullfelddetektor eingesetzt. Jede Abweichung vom Nullfeld, die das SQUID detektiert, wird mit der entsprechenden FLL-Elektronik 1 durch Erzeugen eines Kompensationsfeldes in der Feldaufnahmespule gegengekoppelt. Dieses Signal wird vorzugsweise in der Abschirmung über eine entsprechende Einkoppelspule, die in Serie mit den Feldaufnahmespulen liegt, eingespeist. Mit dieser Methode werden innerhalb des Regelbereiches der Elektronik Magnetfeldverzerrungen minimiert.

Durch Einführen von magnetisch entkoppelten Kammern in die Abschirmung kann das Übersprechen des Gegenkopplungssignales auf das SQUID verhindert werden. Um einen größeren Aussteuerungsbereich der Feldaufnahmespulen 10 im FLL-Modus zu erreichen, kann es sinnvoll sein, nur bis zu einem oder mehreren Flußquanten im SQUID auszuregeln und dann durch Rücksetzen des Integrators das Einziehen von Flußquanten in das SQUID zu induzieren. Die auftretenden Flußquantensprünge müssen zur Auswertung der Messung gezählt werden, die Reglerausgangsgröße gibt dann den Bruchteil eines Flußquantes im SQUID an. Mit dieser Methode kann der Dynamikbereich der SQUID-Regelelektronik erheblich vergrößert werden. Außerdem kann so der Dynamikbereich des zur Auswertung evtl. benötigten A/D-Wandlers relativ klein gehalten werden, da die höherwertigen Bits mit dem Zähler erfaßt werden.

Für ein Gerät ohne aufwendige magnetische Abschirmungen, müssen Störfelder aus der Umgebung (z. B. Netzbrummen und Erdmagnetfeld) am Ort der Feldaufnahmespulen 10 kompensiert werden. Dazu kann zusätzlich ein Referenzgradiometer 8 in einiger Entfernung zum ersten, aber entsprechend symmetrisch zum Erregerfeld positioniert eingesetzt werden. Durch Subtraktion der Ausgangsspannungen beider Gradiometer 8, 10 nach der FLL-Elektronik 1 wird so ein elektronisches Gradiometer höherer Ordnung realisiert. Dieses vorverarbeitete Signal kann dann nach einer entsprechenden über einen Computer gesteuerten Offsetkompensation einem A/D-Wandler zugeführt werden. Dadurch kann der erforderliche Dynamikbereich des A/D-Wandlers reduziert werden. Das Ausgangssignal des Referenzgradiometers 8 kann über einen zweiten äquivalenten Datenerfassungskanal digitalisiert werden, um durch Anwendung spezieller Filteralgorithmen eine weitere Störsignalunterdrückung zu erreichen (Ausgleich von Laufzeitunterschieden, Optimalfilter, Frequenzgangkorrektur usw.).

Eine elektrische Schirmung der SQUIDs und der Zuleitungen ist vorteilhaft (HF-Schirmung). In den meisten Fällen wird die Superisolation des Kryostaten ausreichende Schirmwirkung aufweisen. Mit Hilfe des Vektormagnetometers 9 kann zusätzlich die Richtung von magnetischen Störfeldern gemessen und eine effektivere Störfeldunterdrückung erreicht werden. Weiterhin sind alle Störungen, die durch Aufmagnetisierung von in der Umgebung befindlichen magnetischen Körpern hervorgerufen werden, durch entsprechende Materialauswahl des Meßaufbaus zu vermeiden oder durch entsprechende Kalibriermessungen zu ermitteln und in der Auswertung zu berücksichtigen.

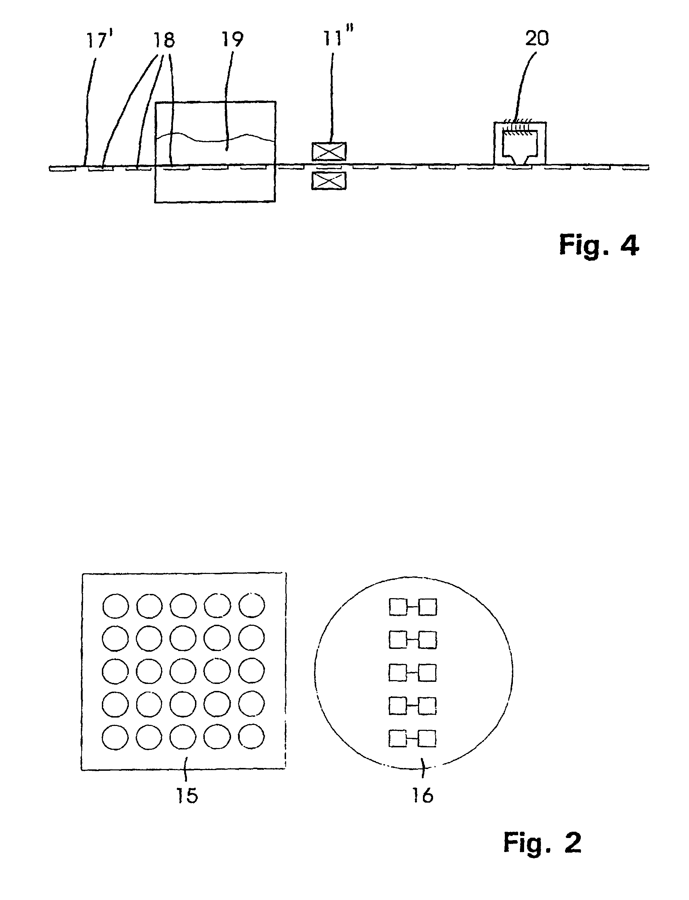

Insbesondere können auch mehrere Proben, die z. B. , wie in Fig. 2 dargestellt, in Form einer Probenmatrix 15 angeordnet sind, gleichzeitig mittels eines mehrkanaligen Meßsystems untersucht werden. Dazu können die Sensoren z. B. in Form eines Arrays oder einer Sensorzeile 16 in einer Ebene angeordnet werden. Diese Meßanordnung ist ebenfalls zur Detektion der räumlichen Verteilung von Analyten geeignet wie es z. B. bei In vivo-Messungen von besonderem Vorteil ist.

Zum Nachweis von Analyten mittels Messung der Relaxation der Magnetisierung von Analyten muß eine schnelle Magnetfeldänderung im Probevolumen gewährleistet werden. Je größer die Feldänderungsgeschwindigkeit, um so kleiner ist die nachweisbare Relaxationszeit.

Ein Meßzyklus kann wie folgt ablaufen:
1) Erzeugung eines Magnetisierungsfeldes mit der Anregungsspule 11. Die Meßprobe 12 sollte sich unter einer der Feldspulen des Sensorgradiometers 10 im Magnetfeld befinden.
2) Abschalten des Magnetfeldes und Messen des am Ausgang der FLL-Regelelektronik 1 entstehenden Signals. Vorteilhaft ist es, wenn das SQUID während des gesamten Vorganges im FLL-Mode betrieben werden kann. Wenn die Änderungsgeschwindigkeit des Magnetfeldgradienten am Sensorgradiometer 10 größer als die Ausgangssignaländerungsgeschwindigkeit der FLL-Elektronik ist, sollte die Regelschleife erst kurze Zeit nach Abschalten des Magnetisierungsfeldes geschlossen werden. Dies kann auch automatisch durch Erreichen des Regelbereiches geschehen.
3) Das Zeitverhalten des SQUID-Ausgangssignales kann nun z. B. durch einen Rechner analysiert werden.
4) Nach Abklingen des transienten Vorganges kann die Prozedur 1) und 2) wiederholt werden, um eine Mittelwertbildung durchführen zu können. Dies kann mit Magnetisierungsfeldern entgegengesetzter Polarität wiederholt geschehen.
5) Gegebenenfalls kann ein weiterer Meßzyklus unter der anderen Feldspule des Sensorgradiometers 10 wiederholt werden.
6) Die nächste Probe kann dann evtl. automatisch unter die Feldaufnahmespulen des Sensorgradiometers positioniert und ausgemessen werden.
7) Es können auch simultane Vergleichsmessungen zwischen zwei Proben durchgeführt werden, indem unter jede Feldspule des Planargradiometers jeweils eine Probe positioniert wird.

Es kann angebracht sein, vor einer Messung einen Kalibrierungszyklus durchzuführen. Dabei wird der Meßzyklus ohne Meßprobe oder mit entsprechenden Eichproben durchgeführt. Die dadurch erhaltene Referenzmessung kann zur Korrektur der Probenmessung verwendet werden.

Zum Nachweis von Analyten mittels Messung der remanenten Magnetisierung kann die Messung der Bindungsremanenz ebenfalls mit dem oben beschriebenen Gerät erfolgen. Ein möglicher Meßablauf wird im folgenden beschrieben:
a) Die in oder unter einer der Feldaufnahmespulen des Sensorgradiometers positionierte Meßprobe wird periodisch mit entgegengesetzter Richtung des Magnetfeldes (bei geringer Frequenz) und gegebenenfalls veränderlicher Amplitude aufmagnetisiert. Vorteilhaft ist die Verwendung eines rampenförmigen zeitlichen Verlaufes der Aufmagnetisierung (Vorteile: maximale Signaländerungsgeschwindigkeit der FLL-Elektronik wird nicht überschritten, Sensorgradiometer kann während des gesamten Meßzyklus im FLL-Modus betrieben werden). Zwischen den Aufmagnetisierungsphasen verbleiben Pausen, in denen die Meßprobe keinem Anregungsfeld ausgesetzt ist.
b) Die FLL-Elektronik verbleibt während de gesamten Meßzyklus im Regelungsmode. Störtransienten werden durch das evtl. erheblich schnellere Referenzgradiometer detektiert und in das Sensorgradiometer direkt zurückgekoppelt.
c) Durch eine geringe Verstimmung des Sensorgradiometers erhält man gleichzeitig ein Maß für die Feldamplitude.
d) In den Magnetisierungspausen wird das von der Probe erzeugte remanente Restfeld gemessen.

Durch die beschriebene Umkehrung des Anregungsfeldes können Störfelddriftprozesse kompensiert werden. Zur verbesserung des Signal-zu-Rausch-Verhältnisses kann die Probe während der Messung bewegt werden (Vibration, Rotation, Ultraschall Hydraulik, Falltür usw.). Dies kann durch unmagnetische Verlängerungen von Hubtischen, Linearmotoren usw. erfolgen. Bei der Konstruktion des Gerätes sollten alle ferromagnetischen Verunreinigungen, die die Meßergebnisse verfälschen können, vermieden werden.

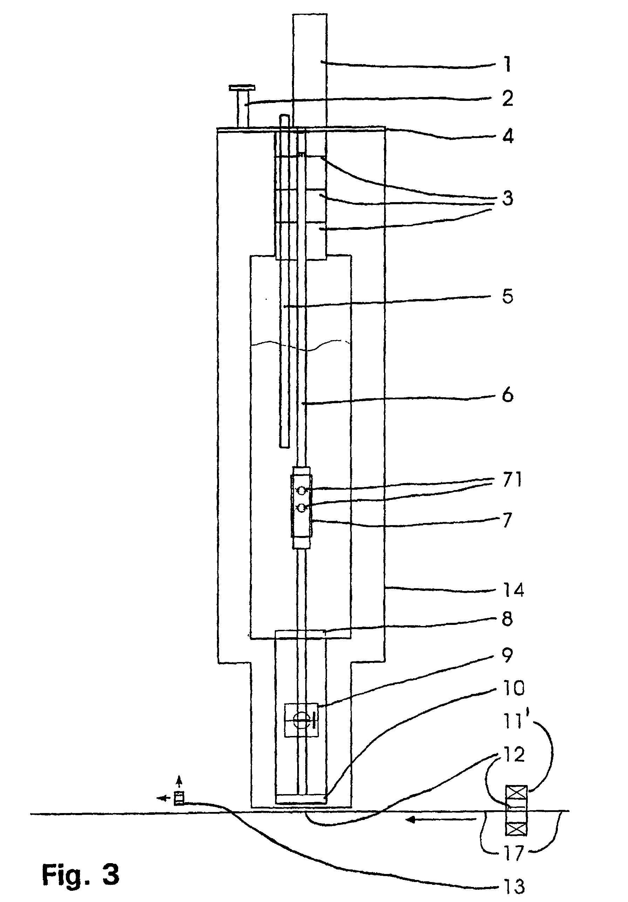

Alternativ zur Aufmagnetisierung der Probe im Meßvolumen kann die Aufmagnetisierung räumlich von der Detektionseinrichtung getrennt erfolgen, wie in Fig. 3 dargestellt ist:

In diesem Fall wird die Meßprobe 12 vom Ort der Aufmagnetisierungseinrichtung, wo sie in der Anregungsspule 11' aufmagnetisiert wird, zum Meßort über einen Mechanismus z. B. mittels Transportband 17 transportiert. Dieser Mechanismus kann gleichzeitig zum Wechseln der Meßproben 12 benutzt werden. Außerdem wird durch den obigen Mechanismus eine Modulation des von der Meßprobe generierten Magnetfeldes am Ort der Feldaufnahmespulen erzeugt.

Weiter können folgende zusätzliche Störfeldunterdrückungsmaßnahmen durchgeführt werden:

Ein dreiachsiges Vektormagnetometer 9 bzw. Vektorgradiometer bestehend aus drei SQUID-Magnetometern, die orthogonal zueinander auf den Seiten eines Würfels angeordnet sind und jeweils im FLL-Modus betrieben werden, kann zur Erzeugung von Referenzsignalen eingesetzt werden. Durch entsprechend gewichtete Subtraktion der Referenzsignale vom Gradiometerausgangssignal wird eine Störunterdrückung erreicht. Dies kann vorteilhaft in zwei Stufen erfolgen. Durch manuellen Abgleich der gewichteten Ausgangssignale des Vektormagnetometers 9 und des Sensorgradiometers 10 wird der Dynamikbereich des Meßsignals für die folgende A/D-Wandlung und Weiterverarbeitung in einem Rechner reduziert. In der zweiten Stufe werden mit Hilfe entsprechender Algorithmen die einzelnen Signale der Vektormagnetometer 9 so kombiniert, daß eine maximale Störunterdrückung des Meßsignales erreicht wird. Dies kann durch angepaßte Optimalfilter, die die bereits vorhandenen Korrelationen zwischen den Signalen berücksichtigen, erreicht werden.

Vor der A/D-Wandlung ist für jedes Signal eine angepaßte Offsetkompensation vorgesehen, um den Dynamikbereich des Wandlers zu optimieren. Legt man das Vektormagnetometer 9 mit kleinen SQUID-Induktivitäten aus, können die Reglerbandbreiten im FLL-Modus mehrere MHz erreichen und auch transiente Störungen ausregeln und kompensieren.

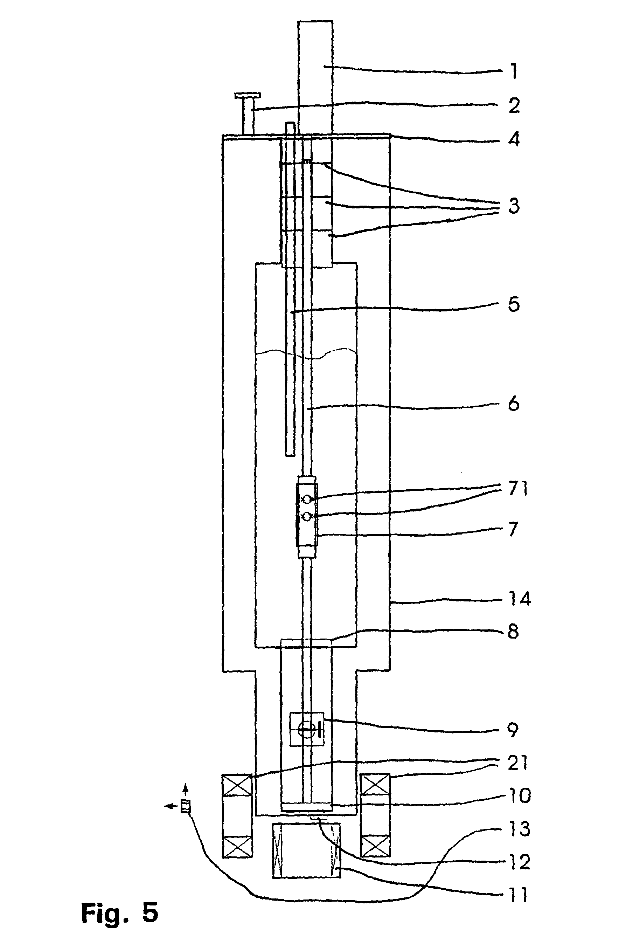

Alternativ zum oben beschriebenen Gerät können auf einem entsprechend präparierten Transportband 17' gebundene remanente Teilchen auch durch Vorbeibewegen des Bandes an einem Magnetfeldsensor 20 (analog zur Magnetbandtechnik) gemessen werden, wie in Fig. 4 dargestellt ist. Das mit z. B. Antigen 18 präparierte Band 17' wird durch ein Bad 19, welches mit remanenten Teilchen markierte Antikörper enthält, bewegt, anschließend durch eine geeignete Anregungsspule 11'' aufmagnetisiert und danach am Magnetfeldsensor 20 vorbeigeführt. Es ist insbesondere vorteilhaft, durch entsprechende periodisch angeordnete Beschichtung des Bandes 17' z. B. mit Antigen 18 eine struktur zu erzeugen, die dazu führt, daß sich Bereiche gebundener remanenter Teilchen mit freien Bereichen derart abwechseln, daß eine definierte Periodizität erzeugt wird.

Bei kontinuierlicher Beschichtung des Bandes 17' kann der obige Effekt auch dadurch erreicht werden, daß das mit den gebundenen remanenten Teilchen präparierte Band durch ein magnetisches Wechselfeld bewegt wird (wiederum analog zur Magnetbandtechnik). Beide oben beschriebenen Vorgehensweisen führen am Magnetfeldsensor 20 zu einem Signal bekannter Frequenz und bindungsabhängiger Amplitude und können vorteilhafterweise z. B. mittels Lock-in Meßtechnik gemessen werden. Die verwendete Technik ist ähnlich einem Tape Recorder.

Zur Messung der komplexen frequenzabhängigen magnetischen Materialeigenschaften kann eine geringfügig gegenüber dem in Fig. 1 beschriebenen Gerät abgewandelte Vorrichtung eingesetzt werden:

Vorteilhaft ist eine Aufmagnetisierungseinrichtung, die ein homogenes Magnetisierungsfeld am Ort der Feldaufnahmespulen des Sensorgradiometers erzeugt. Das homogene Magnetfeld ist vorteilhafterweise entlang der Richtung der geringsten Feldempfindlichkeit der Feldaufnahmespulen gerichtet und kann z. B. mit einer Helmholtzspulenkonfiguration 21 erzeugt werden. Analog zu den oben beschriebenen Kompensationsmaßnahmen kann das Sensorgradiometer auf kleinste Empfindlichkeit gegenüber dem Anregungsfeld abgeglichen werden.

Die Aufmagnetisierungseinrichtung wird mit einem Wechselstrom variabler Frequenz gespeist (Berücksichtigung von Skin-Effekt und frequenzabhängiger Dispersion der Anregungsspule). Die von den Magnetfeldsensoren gemessene zeitabhängige Magnetisierung wird vorteilhafterweise mittels lock-in Technik phasenstarr zur ebenfalls gemessenen anregenden Feldstärke H ausgewertet. Damit kann Betrag und Phase der Magnetisierung M der Meßprobe für die jeweilige Anregungsfrequenz ermittelt werden. Durch Vergleich mit einer Referenzmessung ungebundener Analyten kann somit die Bindung von Analyten hochempfindlich gemessen werden.

Mit dem in Fig. 5 dargestellten Kombinationsgerät kann schließlich die Bindung von Analyten mittels jeder der Methoden I, II und III vermessen werden. Dabei ist zusätzlich eine Helmholtzspule 21 für die Suszeptibilitätsmessung vorgesehen. Ein solches Gerät dient der quantitativen Detektion von Analyten in Flüssig- und Festphasen mittels Relaxationsmessung, Bindungsremanenzmessung sowie mittels frequenzabhängiger komplexer magnetischer Materialeigenschaften.

Die oben beschriebenen Ausführungsformen der erfindungsgemäßen Vorrichtung, insbesondere diejenigen, bei denen die Detektionseinrichtung eine Vorrichtung zur Messung der Bindungsremanenz und/oder zur magnetorelaxometrischen Detektion enthält, können auch speziell für In vivo-Messungen an Menschen oder an Tieren ausgestaltet sein.

## Patentansprüche

1. Vorrichtung zum qualitativen und/oder quantitativen Nachweis von Analyten in einer insbesondere auch biologischen Meßprobe mittels Rezeptor-Ligand-Bindungen mit einer Aufmagnetisierungseinrichtung zur Erzeugung eines Magnetfeldes am Ort der Meßprobe und mit einer Detektionseinrichtung zur Messung von magnetischen Eigenschaften der Meßprobe,
**dadurch gekennzeichnet,**
**daß** die Aufmagnetisierungseinrichtung (11'; 11'') räumlich derart zu der Detektionseinrichtung (71, 8, 9, 10; 16; 20) angeordnet ist, daß das von der Aufmagnetisierungseinrichtung (11'; 11'') am Ort der Aufmagnetisierung erzeugte Magnetfeld am Ort, den die Meßprobe während der Messung einnimmt, um mindestens einen Faktor 10 abgeschwächt ist.

2. Vorrichtung nach Anspruch 1, **dadurch gekennzeichnet, daß** das Magnetfeld um einen Faktor 1000 oder mehr abgeschwächt ist.

3. Vorrichtung nach einem der vorhergehenden Ansprüche, **dadurch gekennzeichnet, daß** eine Vorrichtung (17; 17') zur Bewegung der Meßprobe (12) während der Meßphase der Detektionseinrichtung (71, 8, 9, 10; 16; 20) vorgesehen ist.

4. Vorrichtung zum qualitativen und/oder quantitativen Nachweis von Analyten in einer insbesondere auch biologischen Meßprobe mittels Rezeptor-Ligand-Bindungen mit einer Aufmagnetisierungseinrichtung zur Erzeugung eines Magnetfeldes am Ort der Meßprobe und mit einer Detektinsonseinrichtung zur Messung von magnetischen Eigenschaften der Meßprobe, wobei eine Schalteinrichtung vorgesehen ist, die über eine vorgegebene Zeitdauer während der Meßphase der Detektionseinrichtung (71, 8, 9, 10; 16) das Magnetfeld der Aufmagnetisierungseinrichtung (11) am Ort der Meßprobe (12) abschalten kann, und wobei eine Vorrichtung (17; 17') zur Bewegung der Meßprobe (12) während der Meßphase der Detektionseinrichtung (71, 8, 9, 10; 16; 20) vorgesehen ist.

5. Vorrichtung zum qualitativen und/oder quantitativen Nachweis von Analyten in einer insbesondere auch biologischen Meßprobe mittels Rezeptor-Ligand-Bindungen mit einer Aufmagnetisierungseinrichtung zur Erzeugung eines Magnetfeldes am Ort der Meßprobe und mit einer Detektionseinrichtung zur Messung von magnetischen Eigenschaften der Meßprobe, wobei eine Schalteinrichtung vorgesehen ist, die über eine vorgegebene Zeitdauer während der Meßphase der Detektionseinrichtung (71, 8, 9, 10; 16) das Magnetfeld der Aufmagnetisierungseinrichtung (11) am Ort der Meßprobe (12) abschalten kann, und wobei die Schalteinrichtung eine erste Einrichtung zum Ein- und Ausschalten des in der Aufmagnetisierungseinrichtung (11) erzeugten Magnetfeldes sowie eine zweite Einrichtung zum Ein- und Ausschalten der Detektionseinrichtung (71, 8, 9, 10; 16) umfaßt.

6. Vorrichtung nach einem der vorhergehenden Ansprüche, **dadurch gekennzeichnet, daß** die Detektionseinrichtung eine Vorrichtung zur Messung der Magnetisierung der Meßprobe enthält.

7. Vorrichtung nach einem der vorhergehenden Ansprüche, **dadurch gekennzeichnet, daß** die Detektionseinrichtung eine Vorrichtung zur Messung der Bindungsremanenz des Analyten in der Meßprobe enthält.

8. Vorrichtung nach einem der Ansprüche 4 bis 6, **dadurch gekennzeichnet, daß** die Detektionseinrichtung eine Vorrichtung zur magnetorelaxometrischen Detektion enthält.

9. Vorrichtung nach einem der vorhergehenden Ansprüche, **dadurch gekennzeichnet, daß** die Detektionseinrichtung mindestens ein SQUID (71) als Teil des Magnetfeldsensors enthält.

10. Vorrichtung nach einem der vorhergehenden Ansprüche, **dadurch gekennzeichnet, daß** die Detektionseinrichtung mindestens eine Induktionsspule (20) als Teil des Magnetfeldsensors enthält.

11. Vorrichtung nach einem der Ansprüche 5 bis 10, **dadurch gekennzeichnet, daß** die erste und die zweite Einrichtung unabhängig voneinander schaltbar sind.

12. Vorrichtung nach einem der Ansprüche 5 bis 10, **dadurch gekennzeichnet, daß** die erste Einrichtung mit einer vorgebbaren, starren zeitlichen Korrelation zur zweiten Einrichtung schaltbar ist.

13. Vorrichtung nach einem der Ansprüche 5 bis 12, **dadurch gekennzeichnet, daß** die erste Einrichtung vorgebbare Feldamplituden und Feldpolaritäten des in der Aufmagnetisierungseinrichtung (11; 21) erzeugten Magnetfeldes erzeugen kann.

14. Vorrichtung nach Anspruch 13, **dadurch gekennzeichnet, daß** die erste Einrichtung vorgebbare zeitliche Amplitudenverläufe und vorgebbare zeitliche Polaritätsverläufe des in der Aufmagnetisierungseinrichtung (11; 21) erzeugten Magnetfeldes erzeugen kann.

15. Vorrichtung nach einem der vorhergehenden Ansprüche, **dadurch gekennzeichnet, daß** eine Einrichtung zur elektronischen Störsignalunterdrückung vorgesehen ist.

16. Vorrichtung nach Anspruch 15, **dadurch gekennzeichnet, daß** die Einrichtung zur elektronische Störsignalunterdrückung eine Einheit zur adaptiven Filterung umfaßt.

17. Vorrichtung nach einem der vorhergehenden Ansprüche, **dadurch gekennzeichnet, daß** eine Einrichtung (9) zur Störfeldvektormessung und eine damit verbundene Einrichtung zur entsprechenden Kompensation des mit der Detektionseinrichtung (71, 8, 9, 10; 16; 21) gemessenen Signales und/oder des von der Aufmagnetisierungseinrichtung (11) erzeugten Magnetfeldes vorgesehen ist.

18. Vorrichtung nach einem der vorhergehenden Ansprüche, insbesondere nach den Ansprüchen 7 oder 8, **dadurch gekennzeichnet, daß** die Vorrichtung für In vivo-Messungen ausgestaltet ist.

## Claims

1. Device for the qualitative and/or quantitative detection of analytes in an, in particular, also biological measuring sample by means of receptor-ligand bonds with a magnetization means for generating a magnetic field at the location of the measuring sample and with a detecting means for measuring magnetic properties of the measuring sample,
**characterized**
**in that** the magnetization means (11'; 11") is spatially disposed relative to the detecting means (71, 8, 9, 10; 16; 20) such that the magnetic field generated by the magnetization means (11'; 11") at the location of magnetization is weakened by a factor of at least 10 at the location where the measuring sample is located during the measurement.

2. Device according to claim 1, **characterized in that** the magnetic field is weakened by a factor of 1000 or more.

3. Device according to any one of the preceding claims, **characterized in that** a device (17; 17') is provided for moving the measuring sample (12) during the measuring phase of the detecting means (71, 8, 9, 10; 16; 20).

4. Device for the qualitative and/or quantitative detection of analytes in an, in particular, also biological measuring sample by means of receptor-ligand bonds with a magnetization means for generating a magnetic field at the location of the measuring sample and with a detecting means for measuring magnetic properties of the measuring sample, wherein a switching means is provided which can switch off the magnetic field of the magnetization means (11) at the location of the measuring sample (12) for a predetermined period of time during the measuring phase of the detecting means (71, 8, 9, 10; 16) and wherein a device (17; 17') is provided for moving the measuring sample (12) during the measuring phase of the detecting means (71, 8, 9, 10; 16; 20).

5. Device for the qualitative and/or quantitative detection of analytes in an, in particular, also biological measuring sample through receptor-ligand bonds with a magnetization means for generating a magnetic field at the location of the measuring sample and with a detecting means for measuring the magnetic properties of the measuring sample, wherein a switching means is provided which can switch off the magnetic field of the magnetization means (11) at the location of the measuring sample (12) for a predetermined period of time during the measuring phase of the detecting means (71, 8, 9, 10; 16) and wherein the switching means comprises a first means for switching on and off the magnetic field generated in the magnetization means (11) and a second means for switching on and off the detecting means (71, 8, 9, 10; 16).

6. Device according to any one of the preceding claims, **characterized in that** the detecting means comprises a device for measuring the magnetization of the measuring sample.

7. Device according to any one of the preceding claims, **characterized in that** the detecting means comprises a device for measuring the binding remanence of the analyte in the measuring sample.

8. Device according to any one of the claims 4 through 6, **characterized in that** the detecting means comprises a device for magnetorelaxometric detection.

9. Device according to any one of the preceding claims, **characterized in that** the detecting means comprises at least one SQUID (71) as part of the magnetic field sensor.

10. Device according to any one of the preceding claims, **characterized in that** the detecting means comprises at least one induction coil (20) as part of the magnetic field sensor.

11. Device according to any one of the claims 5 through 10, **characterized in that** the first and second means can be switched independently of each other.

12. Device according to any one of the claims 5 through 10, **characterized in that** the first means can be switched with predetermined rigid temporal correlation to the second means.

13. Device according to any one of the claims 5 through 12, **characterized in that** the first means can generate predetermined field amplitudes and field polarities of the magnetic field generated in the magnetization means (11; 21).

14. Device according to claim 13, **characterized in that** the first means can generate a predetermined temporal amplitude behaviour and predetermined temporal polarity dependences of the magnetic field generated in the magnetization means (11; 21).

15. Device according to any one of the preceding claims, **characterized in that** a means is provided for electronic suppression of disturbing signals.

16. Device according to claim 15, **characterized in that** the device for electronic disturbing signal suppression comprises a unit for adaptive filtering.

17. Device according to any one of the preceding claims, **characterized in that** a means (9) is provided for disturbing field vector measurement and an associated means for corresponding compensation of the signal measured by the detecting means (71, 8, 9, 10; 16; 21) and/or of the magnetic field generated by the magnetization means (11).

18. Device according to any one of the preceding claims, in particular of the claims 7 or 8, **characterized in that** the device is designed for in-vivo measurements.

## Revendications

1. Appareil pour la mise en évidence qualitative et/ou quantitative de composants d'analyse dans un échantillon d'essai en particulier également biologique au moyen de liaisons récepteur-ligand, comportant un dispositif de magnétisation pour générer un champ magnétique à l'emplacement de l'échantillon d'essai et comportant un dispositif de détection pour mesurer les propriétés magnétiques de l'échantillon d'essai,
**caractérisé en ce que**
le dispositif de magnétisation (11' ; 11") est agencé dans l'espace par rapport au dispositif de détection (71, 8, 9, 10; 16; 20) de telle sorte que le champ magnétique généré par le dispositif de magnétisation (11'; 11") à l'emplacement de la magnétisation est affaibli au moins d'un facteur 10 à l'emplacement qu'occupe l'échantillon d'essai pendant la mesure.

2. Appareil selon la revendication 1, **caractérisé en ce que** le champ magnétique est affaibli d'un facteur 1000 ou plus.

3. Appareil selon l'une des revendications précédentes, **caractérisé en ce qu'**il est prévu un dispositif (17 ; 17') pour déplacer l'échantillon d'essai (12) pendant la phase de mesure du dispositif de détection (71, 8, 9, 10;16;20).

4. Appareil pour la mise en évidence qualitative et/ou quantitative de composants d'analyse dans un échantillon d'essai en partzculier également biologique au moyen de liaisons récepteur-ligand, comportant un dispositif de magnétisation pour générer un champ magnétique à l'emplacement de l'échantillon d'essai et comportant un dispositif de détection pour mesurer les propriétés magnétiques de l'échantillon d'essai, dans lequel est prévu un dispositif de commutation qui est capable de couper le champ magnétique du dispositif de magnétisation (11) à l'emplacement de l'échantillon d'essai (12) pendant une période temporelle prédéterminée pendant la phase de mesure du dispositif de détection (71, 8, 9, 10 ; 16), et dans lequel est prévu un dispositif (17 ; 17') pour déplacer l'échantillon d'essai (12) pendant la phase de mesure du dispositif de détection (71, 8, 9, 10; 16 ; 20).

5. Appareil pour la mise en évidence qualitative et/ou quantitative de composants d'analyse dans un échantillon d'essai en particulier également biologique au moyen de liaisons récepteur-ligand, comportant un dispositif de magnétisation pour générer un champ magnétique à l'emplacement de l'échantillon d'essai et comportant un dispositif de détection pour mesurer les propriétés magnétiques de l'échantillon d'essai, dans lequel est prévu un dispositif de commutation qui est capable de couper le champ magnétique du dispositif de magnétisation (11) à l'emplacement de l'échantillon d'essai (12) pendant une période temporelle prédéterminée pendant la phase de mesure du dispositif de détection (71, 8, 9, 10 ; 16), et dans lequel le dispositif de commutation comprend un premier dispositif pour mettre en marche et couper le champ magnétique généré dans le dispositif de magnétisation (11) ainsi qu'un deuxième dispositif pour mettre en marche et couper le dispositif de détection (71, 8, 9, 10 ; 16).

6. Appareil selon l'une des revendications précédentes, **caractérisé en ce que** le dispositif de détection comprend un dispositif pour mesurer la magnétisation de l'échantillon d'essai.

7. Appareil selon l'une des revendications précédentes, **caractérisé en ce que** le dispositif de détection comprend un dispositif pour mesurer la rémanence de liaison du composant d'analyse dans l'échantillon d'essai.

8. Appareil selon l'une des revendications 4 à 6, **caractérisé en ce que** le dispositif de détection comprend un dispositif pour la détection magnéto-relaxométrique.

9. Appareil selon l'une des revendications précédentes, **caractérisé en ce que** le dispositif de détection comprend au moins un SQUID (71) à titre de partie du détecteur de champ magnétique.

10. Appareil selon l'une des revendications précédentes, **caractérisé en ce que** le dispositif de détection comprend au moins une bobine d'induction (20) à titre de partie du détecteur de champ magnétique.

11. Appareil selon l'une des revendications 5 à 10, **caractérisé en ce que** le premier dispositif et le deuxième dispositif sont commutables indépendamment l'un de l'autre.

12. Appareil selon l'une des revendications 5 à 10, **caractérisé en ce que** le premier dispositif est commutable avec une corrélation temporelle fixe prédéterminée par rapport au deuxième dispositif.

13. Appareil selon l'une des revendications 5 à 12, **caractérisé en ce que** le premier dispositif peut générer des amplitudes de champ et des polarités de champ prédéterminées du champ magnétique généré dans le dispositif de magnétisation (11; 21).

14. Appareil selon la revendication 13, **caractérisé en ce que** le premier dispositif peut générer des évolutions d'amplitude temporelles prédéterminées et des évolutions de polarité temporelles prédéterminées du champ magnétique généré dans le dispositif de magnétisation (11; 21).

15. Appareil selon l'une des revendications précédentes, **caractérisé en ce qu'**il est prévu un dispositif pour la suppression électronique de signaux parasites.

16. Appareil selon la revendication 15, **caractérisé en ce que** le dispositif pour la suppression électronique de signaux parasites comprend une unité pour le filtrage adaptatif.

17. Appareil selon l'une des revendications précédentes, **caractérisé en ce qu'**il est prévu un dispositif (9) pour la mesure vectorielle du champ parasite et un dispositif relié à celui-ci pour la compensation correspondante du signal mesuré par le dispositif de détection (71, 8, 9, 10 ; 16 ; 20) et/ou du champ magnétique généré par le dispositif de magnétisation (11).

18. Appareil selon l'une des revendications précédentes, en particulier selon l'une ou l'autre des revendications 7 et 8, **caractérisé en ce que** l'appareil est réalisé pour des mesures in vivo.
